# DEMANDE DE BREVET EUROPEEN

(11) **EP 0 571 283 A1**
(43) Date de publication de la demande: **24.11.1993**
(21) Numéro de dépôt: 93401292.3
(22) Date de dépôt: 19.05.1993
(51) Int. Cl.: A61L 9/015, A61L 9/03

(54) **Procédé et dispositif pour la désinfection de locaux**

(30) Priorité: 22.05.1992 FR 9206292
(71) Demandeur: Giudicelli, Pascal, F-78530 Buc (FR)
(72) Inventeur: Giudicelli, Pascal, F-78530 Buc (FR)
(74) Mandataire: Bonnetat, Christian

(57) **Abrégé**

- La présente invention concerne un procédé et un dispositif pour la désinfection de locaux, et notamment la purification de l'air de ceux-ci.
- Selon l'invention, le dispositif comprend un réceptacle (5) pour une substance de désinfection qui est diffusée sous forme de vapeur, et des moyens de diffusion (6) de ladite substance dans ledit local.

## Description

La présente invention concerne un procédé et un dispositif pour la désinfection de locaux, et notamment la purification de l'air de ceux-ci.

Des études ont montré qu'une désinfection permanente et continue des locaux climatisés notamment à usage professionnel était nécessaire, études présentant par ailleurs une classification quantitative des différents germes rencontrés en atmosphère climatisée. Cette désinfection est jusqu'à présent déjà effectuée dans le domaine des locaux hospitaliers et, d'une manière générale, dans les locaux "médicaux" recevant un public nombreux. Toutefois, cela n'est pas le cas pour les bureaux et les habitations à usage domestique, et d'une manière générale pour tous les locaux à activité non médicale.

La désinfection de locaux (purification de l'air notamment) fait intervenir deux éléments, à savoir une substance de désinfection et un appareil de diffusion.

La substance de désinfection doit avoir pour caractéristiques d'être bactéricide, fongicide et si possible efficace contre l'invasion virale, et d'être non toxique pour ceux qui la respirent. Actuellement, les substances de désinfection existent uniquement sous forme liquide et sont diffusées sous forme d'aérosols. Deux types de telles substances liquides sont disponibles. D'une part, les substances dites "de désinfection terminale" permettent une désinfection totale (stérilisation) du local. Elles désinfectent par voie aérienne les surfaces du local tout en ne mouillant pas ou en ne graissant pas celles-ci, ont un très large spectre d'activité et nécessitent peu de main d'oeuvre. Toutefois, pour être efficaces, il est nécessaire de les utiliser en quantités relativement importantes, de l'ordre de 150 à 300 ml pour une pièce de 30 m³. D'autre part, les substances dites "de désinfection continue" ne permettent pas une stérilisation totale, mais maintiennent une atmosphère assainie dans les normes préconisées de pureté bactériologique grâce à leur pouvoir bactériostatique. De telles substances, se présentant sous forme liquide, peuvent être associées à un parfum (par exemple citron, pin, lavande, verveine) en laissant une odeur résiduelle agréable, tout en éliminant les odeurs désagréables (tabac). Elles sont sans danger pour l'organisme et peuvent être utilisées en présence de l'occupant, en des quantités réduites.

Cependant, le fait d'utiliser ces produits à l'état liquide diffusés sous forme d'aérosols peut inciter à des plaisanteries douteuses ou pire à des actes criminels par remplacement des produits par d'autres. Si cela est peu probable dans le cas d'une désinfection terminale où l'on fait appel à des équipes spécialisées, cela peut se produire lors de l'utilisation quotidienne ou biquotidienne des substances de désinfection continue.

La présente invention a pour but d'éviter ces inconvénients.

A cet effet, le procédé pour la désinfection de locaux, et notamment la purification de l'air de ceux-ci, dans lequel une substance de désinfection est diffusée par un appareil de diffusion, est remarquable, selon l'invention, en ce que ladite substance de désinfection est dans un état permettant sa diffusion dans le local sous forme de vapeur.

Ainsi, on élimine notamment les risques liés à la désinfection quotidienne par des aérosols, puisque la substance de désinfection est alors sous une forme facilement contrôlable.

En particulier, ladite substance de désinfection peut être une substance imprégnant un matériau de support, qui est diffusée par évaporation. Cependant, il est avantageux que ladite substance de désinfection soit une substance solide qui est diffusée dans le local à désinfecter par sublimation.

Bien que la substance de désinfection puisse, le cas échéant, s'évaporer ou se sublimer spontanément, il est avantageux que ladite substance soit diffusée sous l'action d'une ventilation, ou sous l'action d'une source de chaleur.

Par ailleurs, le dispositif pour la désinfection de locaux, et notamment la purification de l'air de ceux-ci, pour mettre en oeuvre le procédé précédemment défini, est remarquable, selon l'invention, en ce qu'il comprend un réceptacle pour une substance de désinfection qui est diffusée sous forme de vapeur, et des moyens de diffusion de ladite substance dans le local.

Selon le mode particulier de diffusion (spontanée, ventilation, source de chaleur), ce réceptacle peut prendre différentes formes.

Ainsi, ledit réceptacle peut être disposé dans un boîtier présentant des ouvertures vers l'extérieur, boîtier dans lequel est logé un ventilateur animé par un moteur électrique.

Ou encore, ledit réceptacle peut être une cuve entourée par une résistance chauffante.

Dans ce dernier cas, bien qu'une forme cubique de la cuve puisse être envisagée, il est avantageux que ladite cuve présente une forme de cône dont la pointe est orientée vers le fond de la cuve.

Dans les deux cas cités, le dispositif comprend, de préférence, une minuterie commandable.

De plus, dans une autre réalisation avantageuse de l'invention, la substance de désinfection se présente sous la forme d'un anneau placé dans une gaine de ventilation, et dans lequel est intégrée une résistance chauffante.

Le dispositif selon l'invention peut être autonome, mais également être intégré dans un système de climatisation et/ou de ventilation, soit au niveau de la gaine d'air de soufflage, soit au niveau du caisson de circulation d'air, notamment.

Les figures du dessin annexé feront bien comprendre comment l'invention peut être réalisée.

La figure 1 montre schématiquement un diffuseur par ventilation pour une substance de désinfection solide selon l'invention , dont on a retiré les faces latérales du boîtier pour la clarté de la représentation.

La figure 2 montre une face latérale du boîtier du diffuseur de la figure 1.

La figure 3 montre la face supérieure du boîtier du diffuseur de la figure 1.

La figure 4 montre, en vue de détail, une ouverture du boîtier du diffuseur de la figure 1.

Les figures 5 et 6 sont, respectivement, une vue de côté et une vue de dessus d'un diffuseur selon l'invention, par action d'une source de chaleur, représenté schématiquement.

Les figures 7 et 8 sont des figures semblables aux figures 5 et 6, illustrant un autre diffuseur par action thermique.

Les figures 9 et 10 illustrent un autre diffuseur sous forme d'anneau à résistance intégrée, logé dans une gaine.

La figure 11 montre schématiquement un système de climatisation et/ou ventilation dans lequel est intégré un diffuseur selon l'invention.

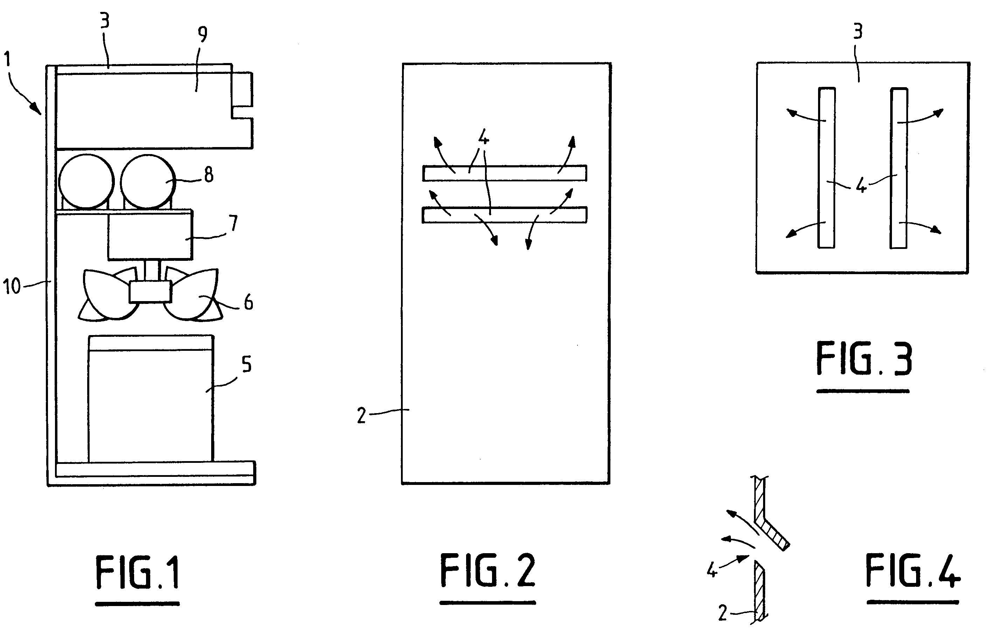

Les figures 1 à 4 illustrent un diffuseur par ventilation selon l'invention comportant un boîtier 1 dont les faces latérales 2 et la face supérieure 3 sont munies d'ouvertures 4 permettant la diffusion, dans l'espace du local à désinfecter, de la substance de désinfection sublimée (donc initialement sous forme solide) contenue dans un réceptacle 5 logé dans le boîtier 1 du diffuseur et agencé sous un ventilateur 6 animé par un moteur électrique 7 relié à une source de courant 8 (pile ou adaptateur 220V). Le diffuseur comporte par ailleurs un système de mise en route comportant une minuterie commandable 9 et une clé (non représentée) qui, dans un sens, ouvre le boîtier et, dans l'autre, met en marche la minuterie. Un support 10 permet de fixer le diffuseur à un mur ou une paroi du local, ou en tout autre endroit approprié.

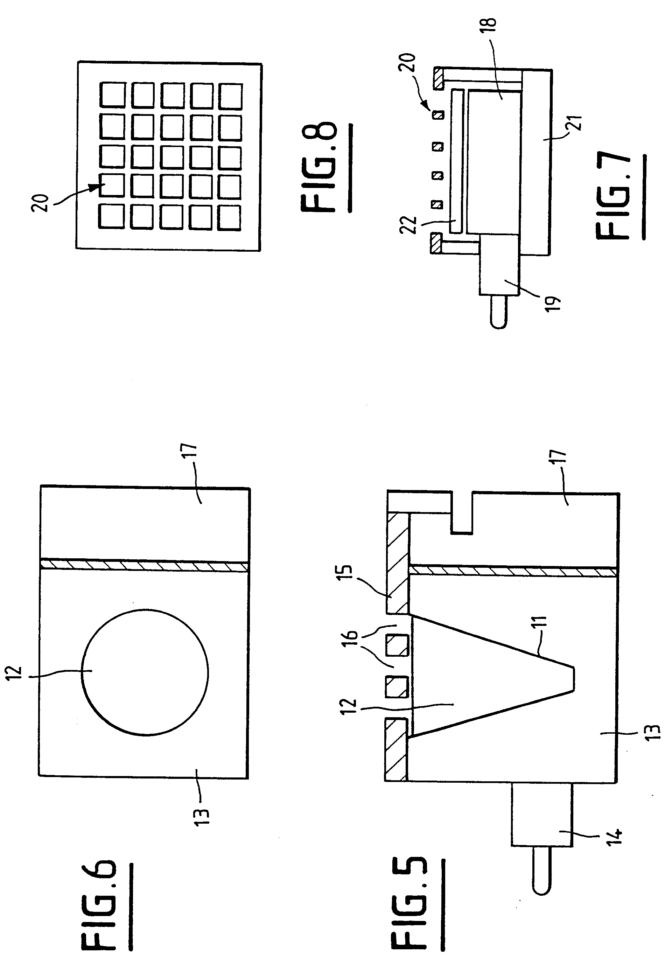

Les figures 5 et 6 montrent un diffuseur à action thermique, comportant une cuve 11, destinée à recevoir la substance, avantageusement solide, de désinfection 12, ladite cuve pouvant présenter une forme cubique ou, avantageusement, une forme de cône dont la pointe est orientée vers le fond de la cuve, comme représenté. Une telle forme, parmi d'autres, garantit que seule la substance de désinfection, présentée sous une forme correspondante, peut être utilisée dans le diffuseur. La cuve 11 est entourée par une résistance chauffante 13 (électrique), par exemple intégrée dans les parois de la cuve, et alimentée en courant par l'intermédiaire d'une prise de courant 14.

La substance de désinfection est sublimée par chauffage et se diffuse à travers un couvercle de diffusion 15 muni d'ouvertures 16. Comme précédemment, le diffuseur comporte un système de mise en route comprenant une minuterie 17 et une clé (non représentée).

Les figures 7 et 8 montrent une variante de réalisation du diffuseur à action thermique des figures 5 et 6, comportant de même une résistance chauffante 18 reliée à une alimentation en courant par l'intermédiaire d'une prise 19, et un couvercle de diffusion 20, le tout étant monté sur un support 21. Dans ce cas, la substance de désinfection peut avoir avantageusement pour support un carton feutre 22 qu'elle imprègne.

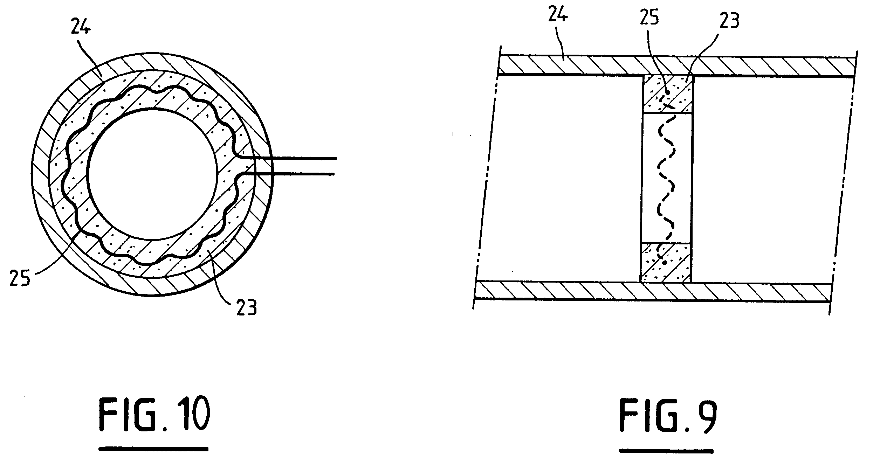

Dans le cas des figures 9 et 10, la substance solide de désinfection se présente sous forme d'un anneau 23, logé dans une gaine de ventilation 24, et dans lequel est intégré une résistance électrique chauffante 25, reliée à une alimentation en courant (non représentée).

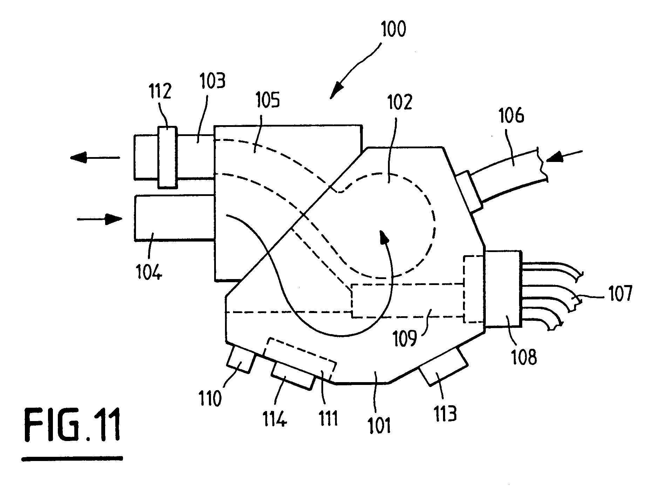

Comme précédemment décrit, les diffuseurs de substance de désinfection selon l'invention peuvent être autonomes, mais ils peuvent être également intégrés dans un système de climatisation et/ou ventilation 100 tel que décrit ci-dessous en référence à la figure 11.

De façon usuelle, le système 100 de climatisation et/ou ventilation comprend un caisson de circulation d'air 101 (flèche) dans lequel est logé un ventilateur 102, et qui est raccordé à une gaine d'air de soufflage 103 et une gaine d'air de reprise 104, par l'intermédiaire d'un caisson de raccordement 105. Par ailleurs, il comporte également une gaine de raccordement d'air extérieur 106, des connexions 107 d'arrivée/retour d'eau chaude/froide, reliées à des vannes 108 de régulation du débit d'eau et une batterie chaude/froide 109. Par ailleurs, il est prévu un module de contrôle électrique 110 et un filtre à air 111.

Dans ce cas, le diffuseur selon l'invention peut être agencé sous forme d'un anneau de substance solide de désinfection 112, analogue à celui montré sur les figures 9 et 10, intégré dans la gaine d'air de soufflage 103. Il peut être également intégré au caisson 101, en se présentant alors sous la forme d'une cuve remplie de produit actif, analogue à la réalisation des figures 5 et 6, et prévue, par exemple, en 113, ou en 114 au niveau du filtre à air 110.

L'utilisation des substances de désinfection varie en fonction des locaux concernés, et peut faire intervenir deux phases. La première phase consiste à stériliser complètement le local. Pour cela, on utilise le produit liquide dit terminal, diffusé par un appareil à aérosol et stérilisant totalement la pièce en 4 à 6 heures selon le cas. En règle générale, le local doit être inoccupé pendant l'opération. L'efficacité extrême de ces produits, mais également les quantités importantes à utiliser, limitent cette phase de stérilisation à une ou deux opérations par an qui doivent être effectuées, comme dans les locaux hospitaliers, par des équipes spécialisées. La seconde phase consiste à prolonger les effets de la première par l'émission, une ou deux fois par jour, de la substance de désinfection continue qui peut être utilisée même en présence de l'occupant du local.

Bien entendu, la nature de la substance de désinfection proprement dite dépend des locaux à désinfecter et des germes que l'on est susceptible d'y rencontrer, ceux-ci étant déterminés par une étude bactériologique préalable. Toutefois, des substances efficaces de ce type contiennent, généralement, des composés tels que le formol, le thymol, le butyl-glycol, l'hexylène-glycol, parmi d'autres. Elles peuvent être utilisées sous forme brute, ou encore comporter des additifs, comme des insecticides.

Par ailleurs, que la substance s'évapore ou se sublime sous l'action d'une ventilation ou d'une source de chaleur, la quantité de celle-ci nécessaire et suffisante pour obtenir la désinfection souhaitée est bien entendu fonction du volume à désinfecter. De plus, le conditionnement "prêt à l'emploi" de la substance dépend du rythme de désinfection, et peut être soit sous forme de dose quotidienne à usage unique, soit sous forme de recharge pouvant être utilisée plusieurs fois.

En outre, le temps de diffusion est fonction de la dose à délivrer (volume de la pièce), et de la puissance du ventilateur en cas de ventilation, ou de la puissance (minimum 5W) et de la surface utile de la résistance électrique en cas d'action thermique.

## Revendications

1. Dispositif pour la désinfection de locaux, et notamment la purification de l'air de ceux-ci, dans lequel une substance de désinfection est diffusée par des moyens de diffusion,
caractérisé en ce qu'il comprend un réceptacle (5,11) servant de support et de contenant de la substance de désinfection qui est diffusée sous forme de vapeur sous l'influence d'une action externe commandable, et en ce que lesdits moyens de diffusion (6,13) de ladite substance dans ledit local sont disposés à proximité directe de ladite substance ou intégrés dans celle-ci ou dans ledit réceptacle.

2. Dispositif selon la revendication 1,
caractérisé en ce que ladite substance de désinfection est une substance liquide imprégnant un matériau de support, qui est diffusée par évaporation.

3. Dispositif selon la revendication 1,
caractérisé en ce que ladite substance de désinfection est une substance solide qui est diffusée dans le local à désinfecter par sublimation.

4. Dispositif selon l'une des revendications 1 à 3,
caractérisé en ce que ladite substance est diffusée sous l'action de moyens de ventilation (6).

5. Dispositif selon l'une des revendications 1 à 3,
caractérisé en ce que ladite substance est diffusée sous l'action d'une source de chaleur (13,18,25).

6. Dispositif selon la revendication 4,
caractérisé en ce que ledit réceptacle (5) est disposé dans un boîtier (1) présentant des ouvertures (4) vers l'extérieur, boîtier dans lequel est logé un ventilateur (6) animé par un moteur électrique (7).

7. Dispositif selon la revendication 5,
caractérisé en ce que ledit réceptacle est une cuve (11) entourée par une résistance chauffante (13).

8. Dispositif selon la revendication 7,
caractérisé en ce que ladite cuve (11) présente une forme de cône dont la pointe est orientée vers le fond de la cuve.

9. Dispositif selon la revendication 6 ou la revendication 7,
caractérisé en ce qu'il comprend une minuterie commandable (9,17).

10. Dispositif selon la revendication 2,
caractérisé en ce que ladite substance imprègne une plaquette (22) au contact direct d'une source de chaleur (18).

11. Dispositif selon la revendication 10,
caractérisé en ce que ladite plaquette est un carton feutre (22).

12. Dispositif selon la revendication 3,
caractérisé en ce que la substance de désinfection se présente sous la forme d'un anneau (23) placé dans une gaine de ventilation (24), et dans lequel est intégrée une résistance chauffante (25).

13. Dispositif selon l'une quelconque des revendications 1 à 12,
caractérisé en ce qu'il est intégré dans un système de climatisation et/ou de ventilation (100).

14. Dispositif selon la revendication 13,
caractérisé en ce qu'il est intégré au niveau de la gaine d'air de soufflage (103).

15. Dispositif selon la revendication 13,
caractérisé en ce qu'il est intégré au niveau du caisson de circulation d'air (101).
